(19) Europäisches Patentamt
European Patent Office
Office européen des brevets

(11) Publication number: **0 411 495 A2**

# (12) EUROPEAN PATENT APPLICATION

(21) Application number: **90114445.1**

(22) Date of filing: **27.07.90**

(51) Int. Cl.[5]: **C07D 457/08**, C07D 457/02, A61K 31/435

(30) Priority: **04.08.89 IT 2145589**

(43) Date of publication of application: **06.02.91 Bulletin 91/06**

(84) Designated Contracting States: **BE DE ES FR GB**

(71) Applicant: **POLI INDUSTRIA CHIMICA S.p.A.**
**Piazza Agrippa, 1**
**I-20141 Milano(IT)**

(72) Inventor: **Poli, Stefano**
**Piazza Agrippa 1**
**I-20141 Milano(IT)**
Inventor: **Coppi, Germano**
**Piazza Agrippa 1**
**I-20141 Milano(IT)**
Inventor: **del Corona, Lucio**
**Piazza Agrippa 1**
**I-20141 Milano(IT)**

(74) Representative: **Minoja, Fabrizio**
**Studio Consulenza Brevettuale Via Rossini, 8**
**I-20122 Milano(IT)**

## (54) Ergoline derivatives of proline and related compounds having dopaminergic activity.

(57) Ergoline derivatives containing the L-proline moiety and some derivatives thereof having dopaminergic activity, the processes for the preparation thereof and pharmaceutical compositions containing them.

EP 0 411 495 A2

## ERGOLINE DERIVATIVES OF PROLINE AND RELATED COMPOUNDS HAVING DOPAMINERGIC ACTIVITY

The present invention relates to compounds of formula (I):

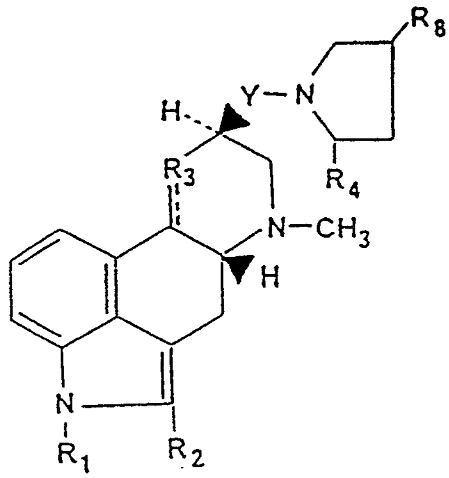

(I)

wherein:
$R_1$ is hydrogen or $C_1$-$C_6$ alkyl;
$R_2$ is hydrogen, chlorine, bromine or iodine;
$R_3$ is CH or $CH_2$, depending on the presence or absence of the double bond represented by the dotted line;
$R_4$ is $COOR_5$, CN, $CH_2OH$, $CONR_6R_7$, wherein $R_5$ is hydrogen or $C_1$-$C_6$ alkyl, $R_6$ and $R_7$ are hydrogen, $C_1$-$C_6$ alkyl, $C_7$-$C_{10}$ aralkyl;
$R_8$ is hydrogen or OH;
Y is CO or $CH_2$.

Preferably, $R_1$ is hydrogen or methyl, $R_2$ is hydrogen or bromine, $R_4$ is COOH, $COOCH_3$, CN, $CH_2OH$, $CONH_2$ and $R_8$ is hydrogen or OH.

The present invention also relates to the pharmaceutically acceptable acid salts of the compounds of formula (I).

In recent years, different lysergic acid derivatives have been synthesized in order to find out dopaminergic agents alternative to Ergot alkaloids obtained by fermentation or extraction from Claviceps purpurea cultures. The main aim of this search is to provide molecules which could selectively act on dopaminergic receptors, reducing the toxic side-effects characteristic of this class of substances.

Ergoline derivatives of formula (I) satisfactorily fulfil said requirements, since they provide to have very effective antihypertensive and antihyperprolactinemic activities and in principle better activity on Central Nervous System than such known drugs as dihydroergocristine, dihydroergocryptine, bromocryptine, nicergoline, metergoline, cabergoline and methysergid.

Particularly, the compounds identified in the examples below by the codes P 1409, P 1410, P 1414, P 1418, P 1422 have a high hypotensive activity, compounds P 1410, P 1419, P 1420, P 1422 show an interesting antihyperprolactinemic activity, compounds P 1409, P 1410, P 1413, P 1415, P 1416, P 1417, P 1418, P 1420, P 1421 and P 1422 have a remarkable antiserotonin activity and compounds P 1409, P 1410, P 1419 and P 1420 a marked activity on C.N.S.

Compounds of formula (I) generally have a marked dopaminergic action at the C.N.S. level.

The results from the pharmacological tests carried out with the compounds of the invention are summarized in the following Tables.

TABLE I -

| Stereotypy test in the rat | | |
|---|---|---|
| Compound | Dose mg/kg i.p. | Mean score |
| P 1409 | 1.25 | 1.00 |
| P 1410 | 10 | 2.62 |
| P 1419 | 0.625 | 2.50 |
| P 1420 | 2.5 | 2.17 |
| α-Bromocryptine | 10 | 2.25 |
| Dihydroergocristine | 10 | 2.37 |

In the locomotor activity in the mouse, all the compounds of the invention displayed a remarkable inhibition, thereby proving the C.N.S. dopaminergic activity.

The results are reported in Table II.

TABLE II

| Compound | Dose mg/kg i.p. | % inhibition |
|---|---|---|
| P 1409 | 2.5 | 69.5 |
| P 1411 | 20 | 80.9 |
| P 1419 | 0.625 | 63.2 |
| P 1420 | 5 | 82.7 |
| P 1422 | 20 | 79.8 |
| α-Bromocryptine | 10 | 78.7 |
| Dihydroergocristine | 10 | 47.7 |
| Dihydro-α-ergocryptine | 20 | 74.3 |

All the compounds of formula (I) have antihypertensive activity on spontaneously hypertensive rats (SHR), as clearly shown by the results reported in the following Table III.

TABLE III

| Compound | Mean arterial pressure % reduction | |
|---|---|---|
| | dose = 1 mg/kg i.p. | dose = 3 mg/kg i.p. |
| P 1409 | 22.1 | 27.5 |
| P 1410 | 17.8 | = = |
| P 1414 | 17.9 | 31.2 |
| P 1418 | 16.1 | = = |
| P 1422 | 18.8 | 40.8 |
| Methylergometrine | 15.7 | 29.4 |
| Dihydro-α-ergocryptine | 18.3 | 29.0 |
| Dihydroergocristine | 18.1 | 32.0 |

Most of the compounds of formula (I) have a marked adrenolytic activity in the in vitro rat seminal

vesicle test. The results are shown in Table IV.

TABLE IV

| Compound | $ED_{50}$ (mcg/ml) |
|---|---|
| P 1409 | 1.02 |
| P 1415 | 0.95 |
| P 1416 | 0.43 |
| P 1417 | 3.52 |
| P 1418 | 0.88 |
| P 1419 | 0.22 |
| P 1420 | 0.50 |
| P 1421 | 2.82 |
| P 1422 | 2.89 |
| Methysergid | 2.48 |

All the compounds of the invention have a marked antiserotonin activity in the in vitro test on isolated rat uterus. The results are reported in Table V.

TABLE V

| Compound | $ED_{50}$ (mcg/ml) |
|---|---|
| P 1409 | 0.010 |
| P 1410 | 0.059 |
| P 1411 | 0.370 |
| P 1413 | 0.004 |
| P 1414 | 0.007 |
| P 1415 | 0.015 |
| P 1416 | 0.003 |
| P 1417 | 0.001 |
| P 1418 | 0.002 |
| P 1419 | 0.073 |
| P 1420 | 0.003 |
| P 1421 | 0.011 |
| P 1422 | 0.014 |
| Dihydro-α-ergocryptine | 0.035 |
| Dihydroergocristine | 0.007 |
| α-Bromocryptine | 0.470 |

All compounds (I) have a marked antihyperprolactinemic activity in the reserpinized rat. The results are reported in Table VI.

TABLE VI

| Compound | Plasmatic prolactin % reduction | |
|---|---|---|
| | After 1 hour (dose 0.25 mg/kg os) | After 2 hours (dose 0.50 mg/kg os) |
| P 1410 | 65.7 | |
| P 1419 | 68.4 | 84.1 |
| P 1420 | 50.1 | |
| P 1422 | 77.1 | |
| Dihydro-α-ergocryptine | 52.4 | 66.1 |
| Dihydroergocristine | 47.4 | |
| α-Bromocryptine | 78.2 | 80.9 |

Moreover, compounds (I) have an effective antiag gregating activity on the in vitro guinea pig platelet aggregation induced by ADP. The results are reported in Table VII.

TABLE VII

| Compound | Dose mcg/ml | Platelet aggregation % reduction |
|---|---|---|
| P 1416 | 20 | 25 |
| P 1421 | 40 | 25 |
| Dihydro-α-ergocryptine | 20 | 25 |
| Dihydroergocristine | 40 | 25 |
| α-Bromocryptine | 80 | 25 |

The compounds of the invention have an acute toxicity by the oral route in the mouse ranging from 400 to 1,600 mg/kg, compounds P 1419 and P 1420 being slightly more toxic.

All the compounds of the invention exhibit depression signs at the level of C.N.S. which can be related to the dopaminergic action.

The present invention also relates to all the industrial aspects connected to the use of compounds of formula (I) for the treatment of Parkinson's disease, cephalalgias, depressions, hyperprolactinemias, hypertension and peripheral and cerebral vascular insufficiencies.

Therefore, the present invention also relates to pharmaceutical compositions containing compounds (I) as the active ingredients, suitably formulated for the oral administration, in form of hard or soft gelatin capsules, dragees, tablets, granulates, drops, syrups, sustained-release forms and for the parenteral administration, in form of solutions or freeze-dried vials.

The pharmaceutical compositions of the invention are prepared according to conventional techniques, using compatible, pharmaceutically acceptable carriers and excipients, and could also contain, in combination, other active ingredients having complementary or anyhow useful activity.

The does will vary depending on the age, body weight and condition of the patient, as well as on the nature and severity of the disorder; generally it will range from 1 to 100 mg of active ingredient, preferably from 20 to 20 mg for the oral forms; from 0.1 to 20 mg, preferably from 0.2 to 5 mg, for the parenteral forms.

The compounds of the invention can be prepared according to conventional methods, starting from lysergic acid, for instance according to the reaction scheme A.

SCHEME A

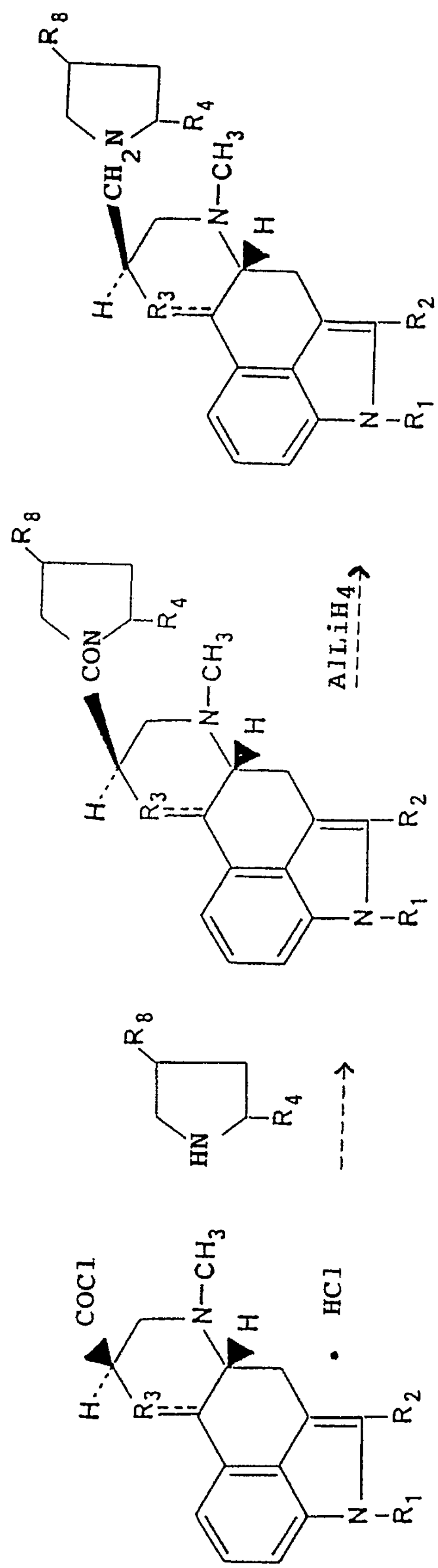


II

I (Y = CO)

I (Y = $CH_2$)

Chloride hydrochloride (II) (GB pat. 1.277.006) is condensed with the corresponding L-proline derivatives in solvents like chloroform, methylene chloride, tetrahydrofuran, dioxane or mixtures thereof, in the presence of organic bases like triethylamine, to give the corresponding amides I (Y = CO) which are then treated with metal hydrides, like $AlLiH_4$, sodium dihydrobis(2-methoxyethoxy)aluminate, in solvents like

tetrahydrofuran, benzene or toluene, to give corresponding compounds (I) (Y = $CH_2$).

The following non limiting examples illustrate the invention in more detail.

## EXAMPLE 1

N-(D-Lysergyl)-L-proline methyl ester maleate (P 1409: I, $R_1$ = $R_2$ = $R_8$ = H, $R_3$ = CH, $R_4$ = $COOCH_3$, Y = CO).

4.55 g (0.033 mole) of methyl L-prolinate are added, during 5 minutes, to a suspension of 2.23 g (0.01 mole) of D-lysergyl chloride hydrochloride in 200 ml of dichloromethane, cooled to 0° C. The reaction mixture is stirred for 30 min. at room temperature, then washed with diluted ammonium hydroxide, dried over anhydrous sodium sulfate and solvent is evaporated off under vacuum. The resulting residue is taken up into 20 ml of methanol and treated with maleic acid, the crystalline precipitate is filtered and washed with ether, to give 2.64 g (53.3%). M.p. 208°-210° C.

Elemental analysis for $C_{22}H_{25}N_3O_3.C_4H_4O_4$

Calc. : H% = 5.90 C% = 63.04 N% = 8.48

Found: H% = 5.98 C% = 62.91 N% = 8.40

## EXAMPLE 2

N-(D-Lysergyl)-4-hydroxy-L-proline methyl ester maleate (P 1413; I, $R_1$ = $R_2$ = H, $R_3$ = CH, $R_4$ = $COOCH_3$, $R_8$ = OH, Y = CO).

4.84 g (0.015 mole) of D-lysergyl chloride hydrochloride are added to a stirred suspension of 2.72 g (0.015 mole) of 4-hydroxy-L-proline methyl ester hydrochloride and 7 ml (0.05 mole) of triethylamine in 50 ml of dioxane and 150 ml of chloroform, cooled to 0° C. After 30 min. at room temperature, water is added, the aqueous phase is extracted with chloroform, the organic phase is washed with diluted ammonium hydroxide, dried over sodium sulfate and concentrated. The concentrated solution is filtered through 100 g of aluminium oxide, the eluate is concentrated, the residue is taken up into methanol and treated with maleic acid. Upon cooling, a product crystallizes which is filtered and dried, to give 5.52 g (72%) of maleate. M.p. 203°-205° C Elemental analysis for $C_{22}H_{25}N_3O_4.C_4H_4O_4$

Calc. : H% = 5.71 C% = 61.06 N% = 8.21

Found: H% = 5.84 C% = 61.01 N% = 8.12

## EXAMPLE 3

N-(D-Lysergyl)-4-hydroxy-L-prolinol (P 1414; $R_1$ = $R_2$ = H, $R_3$ = CH, $R_4$ = $CH_2OH$, $R_8$ = OH, Y = CO).

The base obtained from 5.11 g (0.01 mole) of N-(D-lysergyl)-4-hydroxy-L-proline methyl ester maleate and 4.07 g (0.037 mole) of calcium chloride are dissolved in 500 ml of ethanol and the solution is cooled to -10° C. 2.16 g (0.05 mole) of sodium borohydride are added and the reaction mixture is kept at -5° C for 4 hours, then it is acidified with 1:1 hydrochloric acid, at 0° C, to pH 4-5, solvent is evaporated off, the mixture is alkalinized to pH 8-9, extracted with chloroform. The extract is dried over sodium sulfate and evaporated under vacuum, the residue is taken up into methanol and passed through FLORISIL (50 g), eluting with 3:1 chloroform-methanol. The fractions containing the pure product are pooled and evaporated, to give 2.7 g (73.5%) of the product. M.p. 160-165° C, as solvate with chloroform.

Elemental analysis for $C_{21}H_{25}N_3O_3.CHCl_3$

Calc. : H% = 5.38 C% = 54.27 N% = 8.63

Found: H% = 5.29 C% = 54.21 N% = 8.58

Following the same procedures, starting from the appropriate compounds, the products listed in Table VIII were obtained.

Table VIII

| Compound I | | | | | | | | |
|---|---|---|---|---|---|---|---|---|
| P | FORMULA | $R_1$ | $R_2$ | $R_3$ | $R_4$ | $R_8$ | Y | M.p. °C |
| 1410 | $C_{21}H_{26}N_3O_2.C_4H_4O_4$ | H | H | CH | $CH_2OH$ | H | CO | 110-2 |
| 1411 | $C_{21}H_{23}N_3O_3$ | H | H | CH | COOH | H | CO | 275-8 |
| 1415 | $C_{21}H_{24}BrN_3O_3$ | H | Br | CH | $CH_2OH$ | OH | CO | amorph. |
| 1417 | $C_{22}H_{27}N_3O_3.HCl$ | $CH_3$ | H | CH | $CH_2OH$ | OH | CO | 253-4 |
| 1419 | $C_{21}H_{22}N_4O.HCl$ | H | H | CH | CN | H | CO | 255-7 |
| 1420 | $C_{22}H_{24}N_4O.HCl$ | $CH_3$ | H | CH | CN | H | CO | 268-70 |
| 1421 | $C_{21}H_{27}N_3O.2HCl$ | H | H | CH | $CH_2OH$ | H | $CH_2$ | 252-3 |
| 1422 | $C_{21}H_{27}N_3O_3$ | H | H | $CH_2$ | $CH_2OH$ | OH | CO | 230-5 |
| 1433 | $C_{21}H_{27}N_3O_2$ | H | H | $CH_2$ | $CH_2OH$ | H | CO | 163-5 |
| 1454 | $C_{21}H_{29}N_3O.2HCl$ | H | H | $CH_2$ | $CH_2OH$ | H | $CH_2$ | 265-7 |
| 1423 | $C_{21}H_{24}N_4O_2$ | H | H | CH | $CONH_2$ | H | CO | dec.190 |

## Claims

1. Compounds of formula (I):

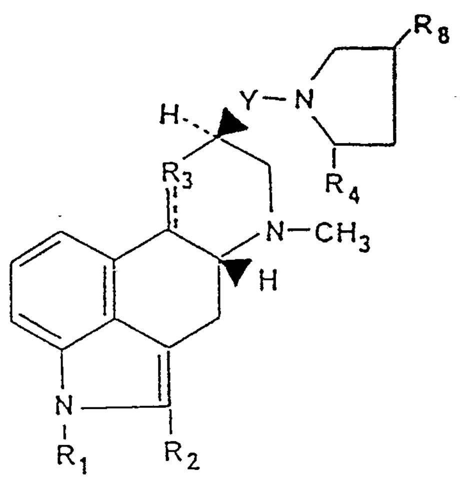

(I)

wherein:
$R_1$ is hydrogen or $C_1$-$C_6$ alkyl;
$R_2$ is hydrogen, chlorine, bromine or iodine;
$R_3$ is CH or $CH_2$, depending on the presence or absence of the double bond represented by the dotted line;
$R_4$ is $COOR_5$, CN, $CH_2OH$, $CONR_6R_7$, wherein $R_5$ is hydrogen or $C_1$-$C_6$ alkyl, $R_6$ and $R_7$ are hydrogen, $C_1$-$C_6$ alkyl, $C_7$-$C_{10}$ aralkyl;
$R_8$ is hydrogen or OH;
Y is CO or $CH_2$; and pharmaceutically acceptable salts thereof.

2. Compounds as claimed in claim 1, wherein $R_1$ is hydrogen or methyl.

3. Compounds as claimed in claims 1 or 2, wherein $R_2$ is hydrogen or bromine.

4. Compounds as claimed in any one of claims 1 to 3, wherein $R_4$ is COOH, $COOCH_3$, CN or $CH_2OH$.

5. Compounds as claimed in any one of claims 1 to 3, wherein $R_8$ is hydrogen or hydroxy.

6. Pharmaceutical compositions containing as the active ingredient one compound as claimed in claims 1 to 5 or a pharmaceutically acceptable salt thereof, in admixture with a pharmaceutically acceptable carrier or excipient.

7. Pharmaceutical compositions as claimed in claim 6, in form of tablets, hard or soft gelatin capsules, sachets, syrup, drops, vials, at doses ranging from 0.1 to 20 mg of active ingredient.

8. A compound as claimed in claims 1 to 5 or a pharmaceutically acceptable salt thereof, for use as a therapeutic agent.

9. The use of a compound as claimed in claims 1 to 5, or of a pharmaceutically acceptable salt thereof, for the preparation of a medicament for the treatment of Parkinsons's disease, cephalalgias, depressions, hyperprolactinemias, hypertension and peripheral and central vascular insufficiencies.